# EUROPEAN PATENT APPLICATION

(11) **EP 4 417 166 A1**
(43) Date of publication of application: **21.08.2024**
(21) Application number: 24157655.2
(22) Date of filing: 14.02.2024
(51) Int. Cl.: A61F 2/46

(54) **DEVICE FOR REMOVING A PROSTHESIS COMPONENT**

(30) Priority: 17.02.2023 IT 202300002739
(71) Applicant: Limacorporate S.p.A., 33038 San Daniele del Friuli (UD) (IT)
(72) Inventor: PRESSACCO, Michele, 33035 Martignacco (UD) (IT); FERRO, Thomas, 33050 Mortegliano (UD) (IT)
(74) Representative: Botti & Ferrari S.p.A.

(57) **Abstract**

Device (101, 201) for removing a prosthesis component, comprising an elongated body (102, 202) having a proximal handle (103) and a distal end (104, 204), a coupling portion (117) at the distal end (104, 204) adapted to couple with the prosthesis component; and at least one cutting element (111, 211) comprising a pivot (124, 224) and a hook-shaped portion (115, 215). The at least one cutting element (111, 211) is movable at least about the pivot (124, 224) between a first position in which the hook-shaped portion (115, 215) is retracted to allow the coupling portion (117) to abut onto the prosthesis component, and a second position in which the hook-shaped portion (115, 215) is adapted to wrap the prosthesis component at least partially. The hook-shaped portion (115, 215) comprises a sharp end (115E, 215E) configured to cut off a bone portion at a rear surface of the prosthesis component.

## Description

### Field of application

The present invention relates to a device for removing a prosthesis component.

In particular, the present invention is mainly but not limitedly directed to a device for removing a glenoid prosthesis component, specifically a baseplate.

The present invention also relates to a removal kit comprising said device and a plurality of adapter elements based on the type of prosthesis component to be removed.

The present invention finds application, in particular in the orthopedic operations which involve the removal of some or all parts of the original prosthesis to replace them with new ones.

### Prior art

In the present technical field, the shoulder prosthesis represents an effective solution in the treatment of joint pain.

In the USA specific shoulder prosthetic surgery was introduced around the 1950s in cases of treatment following serious fractures.

Subsequently, also following the development of prosthetic technology and increasingly safer intervention techniques with increasingly better results, this practice was increasingly also adopted for other painful conditions, such as the various forms of arthrosis.

The number of shoulder prosthetic implants has therefore steadily increased over the last decade.

The types of shoulder prostheses implanted vary depending on the quality of the rotator cuff.

In the absence of a cuff lesion or a divisible lesion, the so-called "anatomical shoulder" is implanted which involves a structure composed of a metal back assembled with an insert. As the name suggests, the design takes the shape of the glenoid cavity.

In case of compromise of the rotator cuff due to irreparable injury, the so-called "reverse shoulder" is implanted, which includes a structure composed of the metal back, a connector and the glenosphere and is characterized by a design opposite the anatomical structure of the shoulder.

The metal back component in contact with the bone may be modular, i.e. made up of the "baseplate" and "peg" subcomponents, or of a monolithic baseplate which combines the "baseplate" and "peg" into a single component.

To have a system suitable even in case of bone deficits, the applicant has also developed a solution for the metal back or baseplate in an increased version, i.e. with different thicknesses along the cross section so as to fill different types of bone deficits in the glenoid and re-establish an optimal condition even in terms of tissue tension.

This solution is described in the international patent application to the Applicant no. WO 2021/099296 A1.

In addition to the external surface of the "peg", the part of the "baseplate" that comes into contact with the bone, called "backside", is also made in such a way as to promote osteointegration with the glenoid and improve the performance of the implant.

Over time, despite the new prosthetic technologies and the new types of interventions, a shoulder prosthesis may be subject to complications for a series of reasons, such as post-operative inconveniences, in the case of infection, periprosthetic fractures, wear of the same prosthesis, etc.

When this happens, a doctor may recommend a second operation to remove some or all of the parts of the original prosthesis and replace them with new ones.

This procedure is called shoulder prosthesis revision.

The revision surgery is different from primary total shoulder arthroplasty as it is a longer and more complex procedure that requires more in-depth preoperative planning and the use of specific prostheses and instruments in order to obtain a good result in terms of pain relief, improvement of the joint function and quality of life.

In some cases, the need may be to specifically remove the baseplate, which may be osteointegrated and therefore firmly fixed to the bone.

In these cases, the problem arises of allowing an easy removal of a prosthesis or one of its components, specifically the baseplate, even if integrated in the bone, also trying to minimize the quantity of bone, and therefore preserving the residual bone for the subsequent revision which may typically involve the implantation of a new device.

Therefore, the technical problem underlying the present invention is to overcome the aforementioned prior art drawbacks.

Specifically, the aim is to provide a device that allows removing a prosthesis component, or an entire prosthesis, thus minimizing the quantity of removed bone.

An object of the invention is also to provide a device suitable for a practical use by a surgeon.

An object of the invention is also to provide a device intuitive to use, namely without requiring specific additional skills or a long apprenticeship for a surgeon who needs to adopt it.

A further object of the invention is to provide a device that always ensures the safety of the surgical operation for the patient.

Finally, an object of the invention is to provide a device that comprises technical solutions that are simple to implement from a production point of view.

### Summary

A solution idea underlying the present invention is to provide a device that allows coupling in a way that adheres as closely as possible to the features of a prosthesis component, specifically a baseplate, so as to carry out a bone resection as limited as possible to what is strictly necessary.

The technical problem is solved by a device for removing a prosthesis component, comprising an elongated body having a proximal handle and a distal end, a coupling portion at the distal end, the coupling portion being adapted to couple with the prosthesis component, and at least one cutting element comprising a pivot and a hook-shaped portion.

The at least one cutting element is movable about the pivot between a first position in which the hook-shaped portion is retracted to allow the coupling portion to abut onto the prosthesis component, and a second position in which the hook-shaped portion is adapted to wrap the prosthesis component at least partially.

The hook-shaped portion comprises a sharp end configured to cut off a bone portion at a rear surface of the prosthesis component.

Preferably, the coupling portion comprises a coupling surface adapted to couple to a glenoid prosthesis baseplate.

However, nothing prevents from also applying a similar solution to other types of prostheses with a suitable geometry.

Advantageously, the present invention allows perfectly coupling to the prosthesis component to be removed, precisely only cutting off the bone portion substantially matching the prosthesis component to be removed.

According to a preferred but non-limiting embodiment, the device comprises at least two pincer-shaped cutting elements opposite and facing each other.

Advantageously, the present solution allows a more precise cutting, thus reducing the quantity of severed bone.

More preferably, the at least two cutting elements comprise a common pivot.

Still more preferably, the at least two cutting elements comprise respective hook-shaped portions having respective sharp ends adapted to tangentially wrap the rear surface of the prosthesis component.

Advantageously, said solution allows making a cutting exactly of the desired geometry since the hook-shaped portions have a concentric profile with the sphere describing the backside of the baseplate and said hook-shaped portions may only rotate about an axis passing through that point, namely an axis centered in the pivot and exiting from the plane on which the hook-shaped portions lie.

From the point of view of minimizing the quantity of bone removed when removing the baseplate, this solution is the best one since it allows perfectly copying the implant's backside using the concentricity, certainly requiring non-negligible radial dimensions of the device.

According to an alternative embodiment, the device comprises a cutting element with a hook-shaped portion.

Preferably, the elongated body comprises a sliding groove and a curved-track shape at the distal end. The device further comprises a pushing rod having a first end adapted to slide in the sliding groove and a second end connected to the pivot of the cutting element and adapted to abut onto the curved track. The first end being adapted to move the cutting element on the curved track, wrapping the prosthesis component at least partially.

Advantageously, the present solution, with a slightly greater quantity of cut bone, allows having a device with overall smaller radial dimensions to be able to be inserted and easily rotated during the surgical operation, for example in the case of small glenoid, still allowing removing less bone than the solutions currently provided in the prior art.

Preferably, the coupling portion comprises an adapter element removably mounted on the elongated body, and the hook-shaped portion is further adapted to wrap said adapter element at least partially.

The adapter element depends on the type of baseplate implanted and on the cutting element, generally rotating, which makes the seat, suitably creating a spherical cutting.

The adapter element, therefore, is a component that couples to the baseplate, for instance with cylindrical coupling, and that allows inserting and positioning the suitable cutting element so that the blades thereof copy the backside of the baseplate and allow obtaining the desired seat, generally a spherical one.

In particular, once the cutting element is coupled to the adapter element, it is rotated and meanwhile pressed against the baseplate so that the kinematics allows the progressive movement of the blade of the cutting element.

The geometry of the adapter element and cutting element is such as to allow the blade of the cutting element to remove the portion of bone integrated close to the backside.

Advantageously, in order to minimize the quantity of bone removed, there will therefore be as many adapter elements as there are baseplate types. Conversely, there will instead be a single cutting element which is compatible with all adapter elements.

Preferably the adapter element comprises a fixing screw to make said adapter element integral with said prosthesis component.

Advantageously, said solution allows a safer coupling between the components and a grater guarantee of accuracy in the surgical procedure.

Preferably, the device according to the invention further comprises at least one actuator element longitudinally sliding and configured to reversibly move said at least one cutting element between the first position and the second position.

Advantageously, said solution ensures an intuitive and effective operation of the device.

Still preferably, the device further comprises at least one spring element associated with the actuator element, the spring element being adapted to be compressed toward the distal end and bring the device into the second position.

In other words, the action of the spring element is connected to the opening and closing action of the cutting element about the prosthesis component.

Preferably, the spring element is made of a helical spring arranged in a longitudinal direction between the handle and the pivot of the at least one cutting element wrap on the actuator element.

The device is therefore normally blocked with the blades open thanks to the action of the helical spring and, by applying a manual push towards the distal end during the rotation of the cutting element about the same longitudinal axis, the movement of the blades up to the end stroke is obtained so as not to forcedly abut with the pin, or "peg", of the prosthesis.

According to a further aspect, the present invention provides a kit for removing prosthetic components, comprising a device according to what has been described above and a plurality of adapter elements suitable for a plurality of prosthetic components.

Advantageously, it is thus possible to provide a single device also coupable to different adapter elements in order to adapt to different needs deriving from different prostheses.

Further features and advantages will become more apparent from the following detailed description of a preferred, but not exclusive, embodiment, of the device and of the instrumentation according to the present invention, with reference to the appended figures given by way of exemplifying and non-limiting example.

### Brief description of the drawings

In these drawings:
- Figure 1 shows a sectional schematic view of a device according to a first embodiment of the present invention;
- Figure 2 shows an exploded view of the device of Figure 1;
- Figure 3 shows a schematic view of a coupling portion comprising an adapter element of the device according to the present invention;
- Figure 4 shows another schematic view of the coupling portion comprising the adapter element of Figure 3 coupled to a baseplate of a glenoid prosthesis;
- Figure 5 shows a view during the operation of the device of Figure 1;
- Figures 6A and 6B show the device of Figure 1 coupled to a baseplate of a glenoid prosthesis with a cutting element having blades in the open configuration and in the closed configuration, respectively;
- Figures 7A and 7B show another schematic view of the device of Figure 1 coupled to a baseplate of a glenoid prosthesis with a cutting element having blades in the open configuration and in the closed configuration, respectively;
- Figure 8 shows a schematic view of a coupling detail between the device of Figure 1 and the baseplate of the glenoid prosthesis;
- Figure 9 shows a sectional schematic view of a device according to a second embodiment of the present invention;
- Figure 10 shows an exploded view of the device of Figure 9;
- Figure 11 shows a schematic view of a coupling detail between the device of Figure 9 and a baseplate of glenoid prosthesis;
- Figures 12A and 12B show the device of Figure 9 coupled to a baseplate of a glenoid prosthesis with a cutting element having blades in the open configuration and in the closed configuration, respectively;
- Figures 13A, 13B, 14A, and 14B show simulations of the evolution of the position of the cutting element's blade during the rotation, for the two embodiments of the device, respectively, both in the case of a completely open cutting element and in the case of a completely closed cutting element;
- Figure 15 shows a variant of the second embodiment of Figure 9.

In the several figures, the same elements will be indicated by the same reference numbers.

### Detailed description

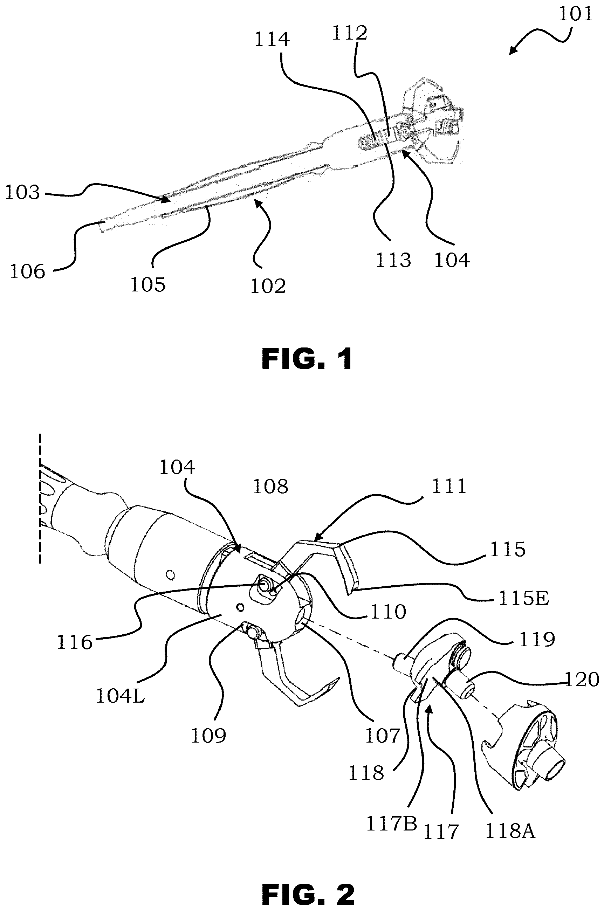

With reference to the enclosed Figures 1-8, reference number 101 globally and schematically indicates a first embodiment of a device for removing a prosthesis component according to the present invention, hereinafter indicated, for the sake of brevity, as device 101.

Specifically, as visible in Figures 1 and 2, the device 101 comprises an elongated body 102 having a proximal handle 103 and a distal end 104.

The proximal handle 103 provides a longitudinal cylindrical development with increasing "stepped" section toward the distal end 104.

Moreover the proximal handle 103 has a grooved outer covering 105 for a firmer grip of the device 1.

Finally, the proximal handle 103 has a proximal end 106.

In a variant, said proximal end 106 may be connected to motorized means (not shown).

The distal end 104 is dome-shaped with respect to the elongated body 102.

The distal end 104 moves integrally with the elongated body 102.

In a possible variant, said dome-shaped distal end 104 may be rotating with respect to the remaining elongated body 102.

At the distal end 104, on the head of the dome, a coupling hole 107, coaxial with the dome-shaped distal end 104, is provided.

On the sides of the coupling hole 107, in the present embodiment, a pair of mutually opposite openings 108, which develop longitudinally on the lateral surface 104L of the dome-shaped distal end 104, is provided.

Furthermore, close to each of said openings 108 a recess 109 wherein there is a slot 110, is provided, the two slots 110 developing in converging directions.

In the present embodiment the device 101 further provides two pincer shaped cutting elements 111 opposite and facing each other.

The cutting elements 111 are moved by the action of an actuator element 112, which is substantially shaped as a rod longitudinally sliding and inserted in a respective cavity 113 that is longitudinally coaxial with the elongated body 112.

A spring element 114A is further associated with said actuator element 112.

In the present embodiment, in particular, a helical spring is provided, which is arranged in a longitudinal direction and wrapped on the actuator element 112.

The device 1 is usually blocked with the cutting elements 111 retracted toward the proximal handle 103 thanks to the action of the spring 114.

Each cutting element 111 comprises a hook-shaped portion 115 having a sharp end 115E.

The expression "hook-shaped" indicates a portion 115 that comprises at least two sections having different longitudinal axis directions.

The cutting elements 111 are mounted inside the distal end 104 and project out of the openings 108.

Moreover, the cutting elements 111 comprise each a lateral pin 116 which is movable inside the slots 110.

In a not represented variant, it would be possible to provide a device comprising a single cutting element 111.

Furthermore, the device 101 comprises a coupling portion 117.

Said coupling portion 117, according to a non-limiting embodiment, comprises an adapter element 117B; more particularly, in the exemplifying and non-limiting represented embodiment, the coupling portion 117 is made of the adapter element 117B, which, still in the present embodiment, is removably mounted on the distal end 104.

Moreover, in other variants, nothing prevents from providing an adapter element 117B made integral with the distal end 104.

The adapter element 117B, in the present embodiment, comprises a main body 118, a first cylindrical projection 119 toward the distal end 104 and adapted to be inserted into the coupling hole 107 and a second cylindrical projection 120 opposite said first projection 119.

Said second projection 120 is adapted to couple with the prosthesis component.

The main body 118 is also shaped so as to be substantially shape-coupled on one side of the distal end 104 and on the other one to the prosthesis component.

In the present description, the provided prosthesis component is a baseplate of a glenoid prosthesis and the main body thus comprises a coupling surface 118A to couple with said baseplate.

However, nothing prevents from applying a solution also similar to other types of prostheses with a suitable geometry.

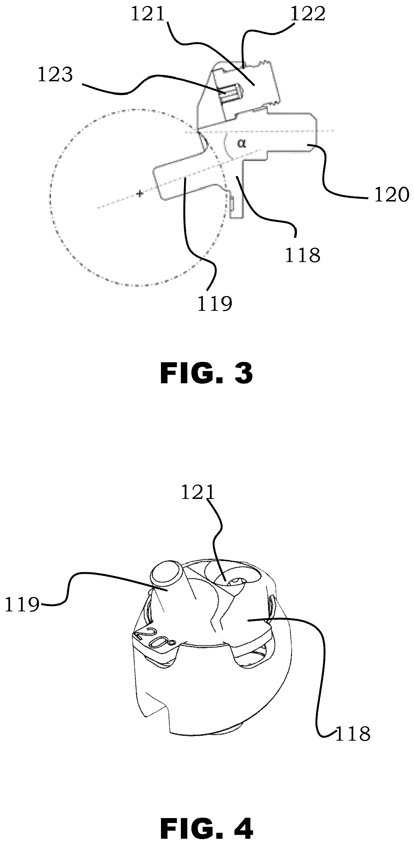

The adapter element 117B, as above stated and as better visible in Figures 3 and 4, is adapted to be coupled though the first projection 119 to the distal end 104 and through the second projection 120 to the prosthesis component. The longitudinal axes of the first projection 119 and of the second projection 120 define an angle α between them to allow the proper coupling according to the arrangement of the prosthesis or of the possible presence of prosthesis components with increased thickness at particular sections.

The angle α thus varies from adapter to adapter.

In the exemplifying and non-limiting embodiment of Figure 4 for instance an angle α equal to 20° is provided.

Moreover, the main body 118 of the adapter 117B comprises a fixing seat 121, generally threaded, adapted to house at least one fixing screw 122 in order to make the adapter element 117B integral with the prosthesis component.

The fixing screw 122 has a screw head 123 through which it is possible to apply the necessary torque. The screw 121 is screwed on the thread of the main body 118 and then remains trapped. The thread itself of the fixing screw 122 is then used to also screw on the baseplate.

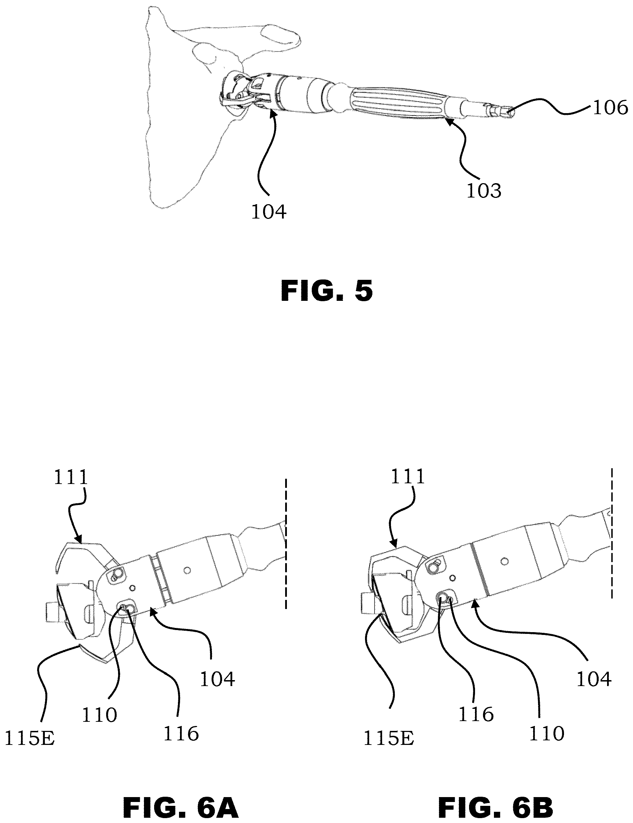

As visible in Figure 5, the device 1 is then brought to abut at the distal end 104 onto the prosthesis component to be removed.

The cutting elements 111 are wrapped, in the present exemplifying and non-limiting embodiment, both on the adapter element 117B and on the prosthesis component, with the hook-shaped portion 115, and in particular the sharp end 115E at the rear surface of the prosthesis component that abuts onto the bone surface.

The sharp end 115E may thus cut off a bone portion at said rear surface of the prosthesis component.

More specifically, as visible in Figures 6A, 6B, 7A, 7B, 8 the cutting elements 111 are movable about a common pivot 124 between a first position, which as above stated is obtained in this embodiment with the action of the spring element 114 wrapped on the actuator element 112, in which the hook-shaped portion is retracted to allow the adapter element 117B, or in general on the coupling portion 117, to abut onto the prosthesis component, and a second position in which the hook-shaped portion is adapted to wrap the prosthesis component at last partially, and in the present case even the adapter element 117B.

Said second position is reached by applying a manual push toward the distal end 104 during a rotation of the cutting elements 111, precisely obtaining the closure of the latter up to the end stroke, however without these abutting against the pin, or "peg", of the baseplate.

The lateral pins 116 of the cutting elements 111 move convergently along the slots 110 stably guiding the passage between the first position and the second position.

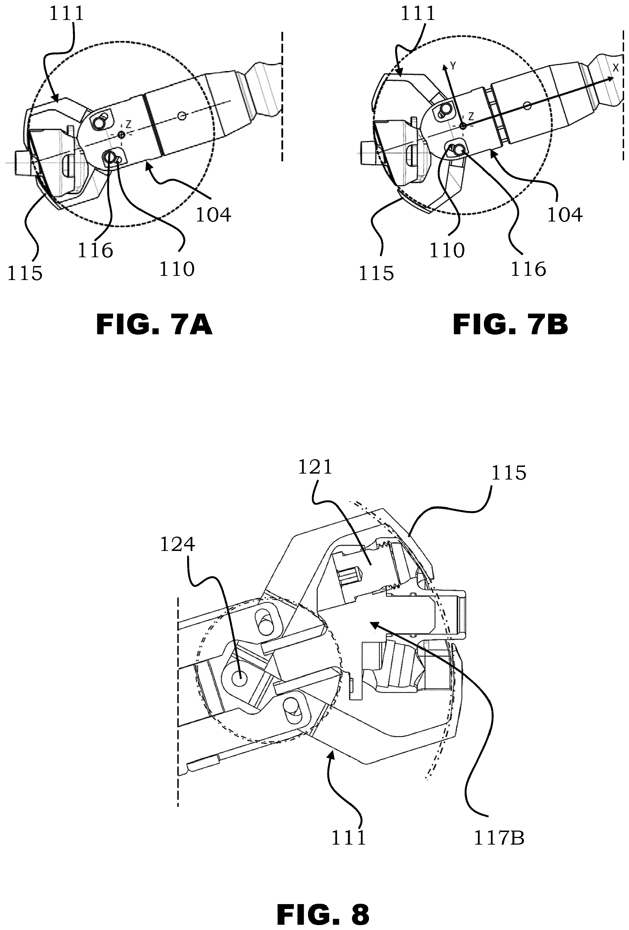

In the present embodiment, as particularly indicated in Figures 7A, 7B, each section of the hook-shaped portion 115 having the sharp end 115E is adapted to tangentially wrap the rear surface of the prosthesis component, in this case of the rear surface of the glenoid baseplate.

In other words, in the present exemplifying embodiment, the sections of the hook-shaped portions 115 comprising the sharp ends 115E, describe a concentric profile with a sphere that in turn also describes the rear surface, or "backside", of the baseplate and said portions 115 may only rotate about an axis passing through the centre of said sphere.

In the sectional view in Figure 8 of the distal end 104, it is still greatly highlighted how the above described concentricity allows perfectly coupling the "backside" of the baseplate, thus minimizing the quantity of resected bone.

The device 101, having said feature, requires specific radial dimensions.

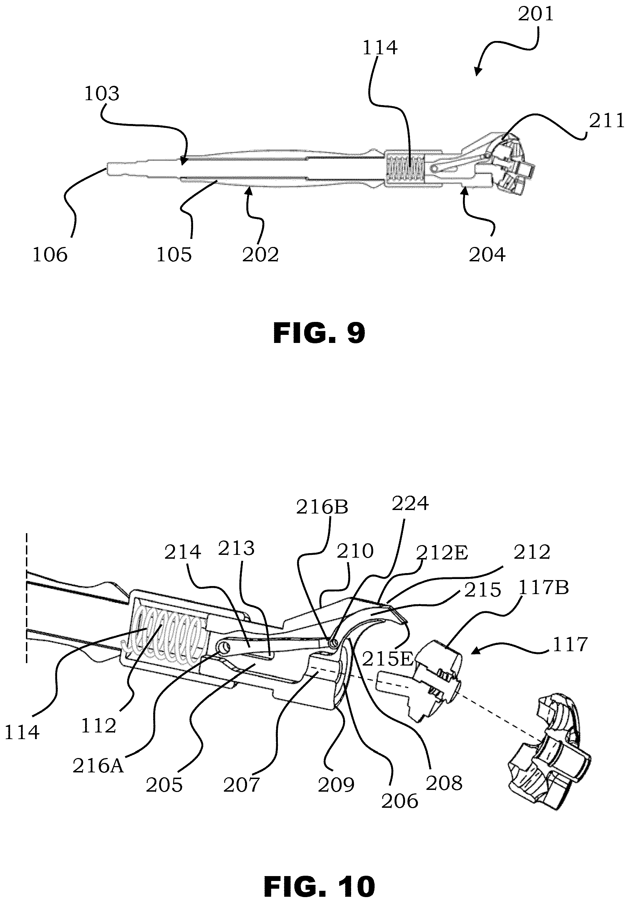

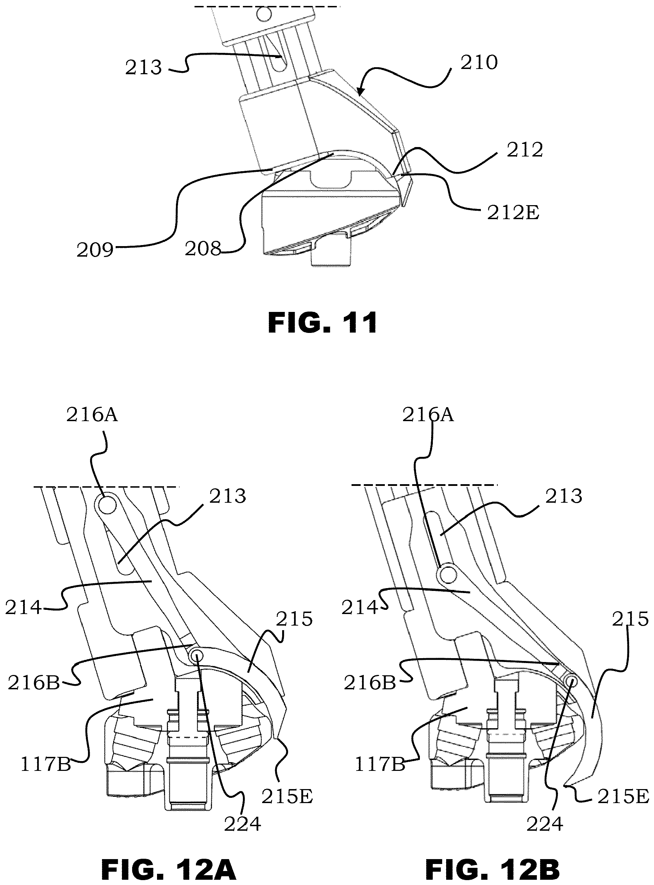

Instead, should it be desired to have more compact dimensions, the present invention also provides a second embodiment, shown in Figures 9-11, of a device for removing a prosthesis component 201, or device 201, which has a single cutting element 211 having a hook-shaped portion 215 with a sharp end 215E.

Hereinafter, we will refer to elements similar to the first embodiment by adopting the same reference numbers.

The hook-shaped cutting element 211, in the described exemplifying and non-limiting embodiments, is different from the hook-shaped element 111 of the first embodiment.

Specifically, the cutting element 211 includes the hook-shaped portion 215 with a less arched conformation, therefore the sharp end 215E is adapted to incise and cut off the bone underlying the prosthesis component less close to the backside of the baseplate.

In other words, a greater quantity of bone will be cut off compared to the first embodiment.

In a not represented variant, it would be possible to provide a device comprising two or more cutting elements 211, preferably opposite each other.

Conversely to the previous embodiment, the present one provides an elongated body 202 comprising a distal end 204.

The distal end 204, in the present embodiment, comprises an internal cavity 205 and a bottom 206 comprising a central coupling hole 207 adapted to house the first projection 119 of the adapter 117B.

The bottom 206 comprises a first circular sector 208 having a substantially cylindrical longitudinal development and a second circular sector 209 having a curved track-shaped longitudinal development and projecting toward the adapter element 117B.

Moreover, the distal end 204 provides a lateral surface 210 having a conformation substantially mirroring that of the bottom 206, with a second hole 212 thus generated at the terminal end 212E of the second circular sector 209.

In the internal cavity 205 of the distal end 204 a sliding groove 213 is provided.

Moreover the device 201 comprises a pushing rod 214.

The pushing rod 214 comprises a first end 216A constrained to slide in the sliding groove 213.

The pushing rod 214 then comprises a second end 216B connected to a pivot 224 of the cutting element 211.

The second end 216B abuts onto the second circular curved track-shaped sector 209.

In this way, further to a sliding of the first end 216A toward the coupling portion 117, in this case the adapter element 117B, the second end 216B travels the path defined by the second circular sector 209, making the cutting element 211 project from the second hole 211 and wrap the prosthesis component at least partially, and in this case even the adapter element 117B.

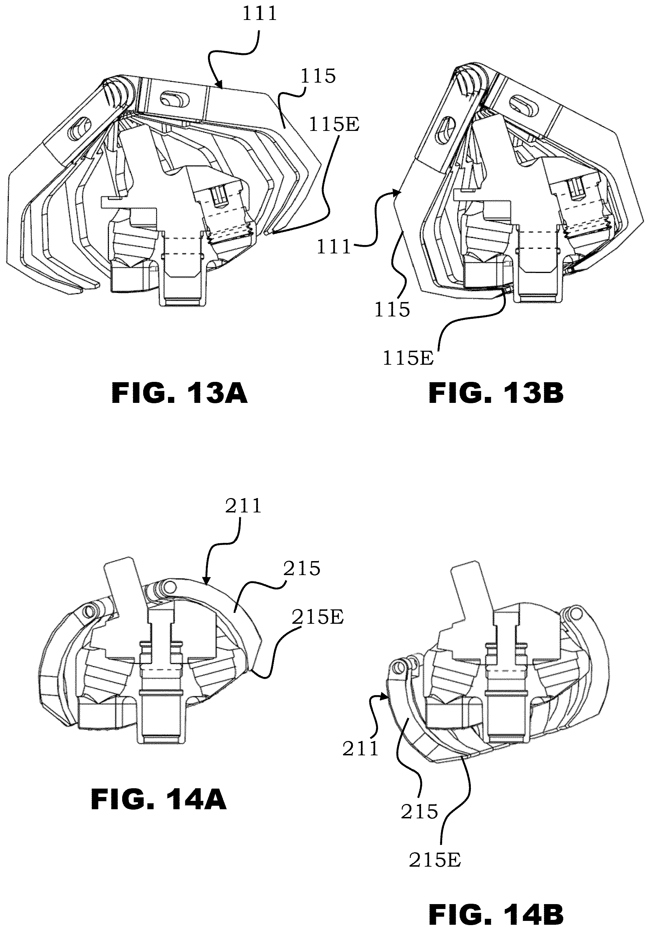

In Figure 13A, 13B and 14A, 14B it is possible to note the development during the rotation of the two above described embodiments both in the first retracted position and in the second position wrapping the adapter element 117B and the prosthesis component at least partially.

Specifically, it is possible to more precisely highlight the several features and the related several advantages of the two embodiments, namely the possibility given by the first embodiment to cut off the minimum bone portion possible for removing the prosthesis component, and the possibility given by the second embodiment to have a more compact and easy to handle tool against a greater quantity of resected bone compared to the first embodiment but less compared to the prior art solutions nowadays available.

The present invention, in the embodiment comprising an adapter element 117B as coupling portion 117, is further addressed to a kit for removing prosthesis components, comprising a device according to what has been described above and a plurality of adapter elements as those above illustrated by way of non-limiting example or other adapter elements, each selected for a specific prosthesis component to be removed.

Advantageously, therefore, the present embodiment allows adopting a same removal device by changing the adapter element, so as to select the one more adherent to the features of the prosthesis component to be removed, both optimizing the removal execution and facilitating the surgeon's task.

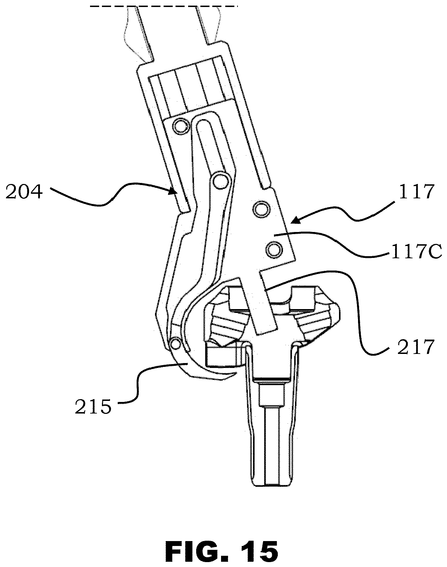

In Figure 15 a variant of the second embodiment is further represented, in which the coupling portion 117 is made of an integral element 117C at the distal end 204 and which has a projecting portion 217 adapted to directly couple to the prosthesis component without interposition of an adapter element 117B, still allowing the hook-shaped portion 215, in its second position, to wrap said prosthesis component at least partially.

The same solution may also be adopted and adapted to the first embodiment above described.

Advantageously and in general, the present invention relates to a device able to reduce the quantity of resected bone in the prosthesis revision procedures, with positive impacts both in the success of the operation and in the timing of recovery of subsequent mobility and physiotherapy.

Still advantageously, the present invention relates to a device that is intuitive to use for a surgeon, who therefore does not need particular practical sessions to be able to use this new tool.

Still advantageously, the device according to the invention has technical features that do not have particular complications from a production point of view, with a positive impact in terms of production times and costs.

Finally, advantageously, the device according to the invention has a robust structure that can ensure patient safety in its use.

It will be clear for a person skilled in the art that several changes and variants can be made to the present invention, all of which fall within the scope of the invention defined by the attached claims.

For instance, with the further research development, it is also possible to develop different shapes of the cutting element with a hook-shaped portion, suitable for contingent peculiarities, these variants being included in the aforementioned scope defined by the attached claims.

## Claims

1. Device (101, 201) for removing a prosthesis component, comprising:
- an elongated body (102, 202) having a proximal handle (103) and a distal end (104, 204);
- a coupling portion (117) at said distal end (104, 204) adapted to couple with said prosthesis component; and
- at least one cutting element (111, 211) comprising a pivot (124, 224) and a hook-shaped portion (115, 215);
wherein said at least one cutting element (111, 211) is movable at least about said pivot (124, 224) between a first position in which said hook-shaped portion (115, 215) is retracted to allow said coupling portion (117) to abut onto said prosthesis component, and a second position in which said hook-shaped portion (115, 215) is adapted to wrap said prosthesis component at least partially,
said hook-shaped portion (115, 215) comprising a sharp end (115E, 215E) configured to cut off a bone portion at a rear surface of said prosthesis component.

2. Device (101) according to claim 1, comprising at least two pincer-shaped cutting elements (111) opposite and facing each other.

3. Device (101) according to claim 2, wherein said at least two cutting elements (111) comprise a common pivot (124).

4. Device (101) according to claim 2 or 3, wherein said at least two cutting elements (111) comprise respective hook-shaped portions (115) having respective sharp ends (215E) adapted to wrap said rear surface of said prosthesis component tangentially.

5. Device (201) according to claim 1, comprising a cutting element (211) with a hook-shaped portion (215).

6. Device (201) according to claim 5, wherein
said elongated body (202) comprises a sliding groove (213) and a curved-track shape at said distal end (204); and wherein
said device (201) further comprises:
a pushing rod (214) having:
- a first end (216A) adapted to slide in said sliding groove (213) and
- a second end (216B) connected to said pivot (224) of said cutting element (211) adapted to abut onto said curved track,
said first end (216A) being adapted to move said cutting element (211) on said curved track, wrapping said prosthesis component at least partially.

7. Device (101, 201) according to any one of claims 1 to 6, wherein said coupling portion (117) comprises an adapter element (117B) removably mounted on said elongated body, and wherein said hook-shaped portion (115, 215) is further adapted to wrap said adapter element (117B) at least partially.

8. Device (101, 201) according to claim 7, wherein said adapter element (117B) comprises a fixing screw (122) adapted to make said adapter element (117B) integral with said prosthesis component.

9. Device (101, 201) according to any one of claims 1 to 8, further comprising at least one actuator element (112) longitudinally sliding and configured to reversibly move said at least one cutting element (111, 211) between said first position and said second position.

10. Device (101, 201) according to claim 9, further comprising at least one spring element (114) associated with said actuator element (112), said spring element (112) being adapted to be compressed toward said distal end (104, 204) and to bring said device (101, 201) into said second position.

11. Device (101, 201) according to any one of claims 1 to 10, wherein said coupling portion (117) comprises a coupling surface (118A) adapted to couple to a glenoid prosthesis baseplate.

12. Kit for removing prosthetic components, comprising a device (101, 201) according to claim 7 or 8 and a plurality of adapter elements (117B) adapted for a plurality of prosthetic components.
